# EUROPEAN PATENT SPECIFICATION

(11) **EP 0 969 860 B1**
(45) Date of publication and mention of the grant of the patent: **17.07.2002**
(21) Application number: 98907914.0
(22) Date of filing: 20.03.1998
(51) Int. Cl.: A61K 38/28

(54) **METHOD FOR PREPARATION OF A THERAPEUTIC POWDER THROUGH COPRECIPITATION OF INSULIN AND ABSORPTION ENHANCER**
VERFAHREN ZUR HERSTELLUNG EINES THERAPEUTISCHEN PUDERS DURCH COPRÄZIPITATION VON INSULIN UND EINEM ABSORPTIONSVERSTÄRKER
PROCEDE DE PREPARATION D'UNE POUDRE THERAPEUTIQUE PAR CO-PRECIPITATION D'INSULINE ET D'UN ACTIVATEUR DE L'ABSORPTION

(30) Priority: 20.03.1997 DK 31897
(43) Date of publication of application: 12.01.2000
(73) Proprietor: NOVO NORDISK A/S, 2880 Bagsvaerd (DK)
(72) Inventor: JENSEN, Steen, DK-2791 Drag r (DK); HANSEN, Philip, DK-2840 Holte (DK)
(74) Representative: Kellberg, Lars
(86) International application number: DK9800107
(87) International publication number: WO9842367

(56) References cited:
- EP-A- 0 692 489
- WO-A-95/00151
- WO-A-95/00550

## Description

### Field of the invention

The present invention relates to a process for the preparation of a therapeutic powder formulation comprising particles composed of insulin or an analogue or derivative thereof and an enhancer which enhances the absorption of insulin in the lower respiratory tract.

### Background of the invention

Diabetes is a general term for disorders in man having excessive urine excretion as in diabetes mellitus and diabetes insipidus. Diabetes mellitus is a metabolic disorder in which the ability to utilize glucose is more or less completely lost. About 2 % of all people suffer from diabetes.

Since the introduction of insulin in the 1920's, continuous strides have been made to improve the treatment of diabetes mellitus. To help avoid extreme glycaemia levels, diabetic patients often practice multiple injection therapy, whereby insulin is administered with each meal.

In the treatment of diabetes mellitus, many varieties of insulin preparations have been suggested and used, such as regular insulin, Semilente® insulin isophane insulin, insulin zinc suspensions, protamine zinc insulin and Ultralente® insulin. Some of the commercial available insulin preparations are characterized by a fast onset of action. Ideally, exogenous insulin is administered at times and in doses that would yield a plasma profile which mimics the plasma profile of endogenous insulin in a normal individual. Insulin preparations containing analogs of human insulin have shown an absorption profile very close to the normal plasma profile, e.g. Lys^{B28}-Pro^{B29} human insulin and Asp^{B28} human insulin. However, these new insulin preparations still has to be injected in connection with a meal. In order to circumvent injections, administration of insulin via the pulmonary route could be an alternative elucidating absorption profiles which mimic the endogenous insulin without the need to inject the insulin.

### Description of the background art

Administration of insulin via the pulmonary route can be accomplished by either an aqueous solution or a powder preparation. A description of the details can be found in several references, one of the latest being by Niven, Crit. Rev. Ther. Drug Carrier Sys, 12(2&3):151-231 (1995). One aspect covered in said review is the stability issue of protein formulations, aqueous solutions being less stable than powder formulation. So far, all powder formulations have been described as mainly amorphous.

EP 692 489 discloses a method for producing crystals of the insulin analogue Lys^{B28}Pro^{B29} human insulin which comprises providing an acidic solution of the insulin analogue, adjusting the pH to 5.5-6.5, and collecting the crystals by decanting the water. The crystals are described as a stable, solid form of the drug substance suitable for holding and dispensing to fill/finish operations.

WO 95/00151 is concerned with a method of increasing absorption of a compound (such as insulin) into the circulatory system by administering e.g. via the inhalation route the compound together with a suitable non-toxic, non-ionic alkyl glycoside having a hydrophobic alkyl joined by a linkage to a hydrophilic saccharide (absorption increasing substance).

It has been found that when insulin is combined with an appropriate absorption enhancer and is introduced into the lower respiratory tract in the form of a powder of appropriate particle size, it readily enters the systemic circulation by absorption through the layer of epithelial cells in the lower respiratory tract as described in US patent No. 5,506,203. The manufacturing process described in said patent, comprising dissolution of insulin at acid pH followed by a pH adjustment to pH 7.4 and addition of sodium taurocholate before drying the solution by a suitable method, results in a powder composed of human insulin and absorption enhancer in a ratio between 9:1 to about 1:1. The powder is characterized as mainly amorphous determined under a polarized light microscope.

WO 96/19207 relates to a powder formulation of medically useful polypeptides such as insulin. The powder formulation is characterised by containing melezitose as diluent, and can furthermore comprise an enhancer which enhances the absorption of the polypeptide in the lower respiratory tract such as sodium taurocholate. Two different general methods for producing powder formulations are disclosed in this document. The first method involves dry mixing of the components as well as micronisation. The second method involves dissolution of the components in a suitable solvent (such as water) and, if desired, adjustment of pH. The powder is then obtained by removing the water by a suitable drying method (i.e. forced precipitation) such as vacuum concentration, open drying, spray drying, and freeze drying.

WO 95/00128 is concerned with dry powder compositions comprising a pharmaceutically active polypeptide and an enhancer which enhances the absorption in the lower respiratory tract. The polypeptide can be a wide range of peptide hormones, but the polypeptide is preferably not insulin.

### Description of the invention

### Definitions

The expressions "crystalline" and "amorphous" as used herein corresponds to different states of a powder particle, distinguishable by the following method: An aliquot of particles of the powder are mounted in mineral oil on a clean glass slide. Examination using a polarized light microscope elucidates birefringence for crystalline particles.

The expression "enhancer" refers to a substance enhancing the absorption of insulin, insulin analogue or insulin derivative through the layer of epithelial cells lining the alveoli of the lung into the adjacent pulmonary vasculature, i.e. the amount of insulin absorbed into the systemic system is higher than the amount absorbed in the absence of enhancer.

By "analogue of human insulin" (or similar expressions) as used herein is meant human insulin in which one or more amino acids have been deleted and/or replaced by other amino acids, including non-codeable amino acids, or human insulin comprising additional amino acids, i.e. more than 51 amino acids.

By "derivative of human insulin" (or similar expressions) as used herein is meant human insulin or an analogue thereof in which at least one organic substituent is bound to one or more of the amino acids.

In the present context the expression "powder" refers to a collection of essentially dry particles, i.e. the moisture content being below about 10 % by weight, preferably below 6 % by weight, and most preferably below 4 % by weight.

According to the invention it has surprisingly been found that it is possible to obtain particles composed of both insulin and enhancer despite the fact that enhancers and particularly surface active agents normally inhibits precipitation.

Thus, the present invention relates to a process for the preparation of a therapeutic powder formulation comprising particles composed of insulin or an analogue or derivative thereof and an enhancer which enhances the absorption of insulin in the lower respiratory tract, comprising the steps of:
a) providing an acidic aqueous solution comprising insulin or an analogue or derivative thereof, an enhancer and optionally zinc;
b) adjusting the pH value to 4.5 to 7.4, preferably 4.5 to 7, more preferably 4.5 to 6.5, still more preferably 5.5 to 6.2, most preferably 5.5 to 6.1;
c) precipitating a product comprising insulin or an analogue or derivative thereof, an enhancer and optionally zinc; and
d) removing the water.

The process of the invention results in a powder formulation of insulin and enhancer which elucidates a better stability profile than powders of essentially the same composition prepared by spray drying, freeze-drying, vacuum drying and open drying. By this means it is possible to store the powder formulations of the present invention at room temperature in contrary to human insulin preparations for injections and some amorphous insulin powders without stabilizing agent which have to be stored between 2°C to 8°C.

Furthermore, the obtained powder formulations elucidates better flowing properties than corresponding powder formulations disclosed in the prior art.

The enhancer is advantageously a surfactant, preferably selected from the group consisting of salts of fatty acids, bile salts or phospholipids, more preferably a bile salt.

Preferred fatty acids salts are salts of C₁₀₋₁₄ fatty acids, such as sodium caprate, sodium laurate and sodium myristate.

Lysophosphatidylcholine is a preferred phospholipid.

Preferred bile salts are salts of ursodeoxycholate, taurocholate, glycocholate and taurodihydrofusidate. Still more preferred are powder formulations according to the invention wherein the enhancer is a salt of taurocholate, preferably sodium taurocholate.

The preferred analogues of human insulin are fast-acting insulin analogues, in particular analogues wherein position B28 is Asp, Lys, Leu, Val or Ala and position B29 is Lys or Pro; or des(B28-B30), des(B27) or des(B30) human insulin. The most preferred analogues are Asp^{B28} human insulin or Lys^{B28}Pro^{B29} human insulin.

The preferred derivatives of human insulin are derivatives comprising one or more lipophilic substituents. The preferred lipophilic insulins are acylated insulins such as those described in WO 95/07931, e.g. human insulin derivatives wherein the ε-amino group of Lys^{B29} contains an acyl substituent which comprises at least 6 carbon atoms.

The insulin derivative is most preferably selected from the group consisting of B29-N^{ε}-myristoyl-des(B30) human insulin, B29-N^{ε}-palmitoyl-des(B30) human insulin, B29-N^{ε}-myristoyl human insulin, B29-N^{ε}-palmitoyl human insulin, B28-N^{ε}-myristoyl Lys^{B28} Pro^{B29} human insulin, B28-N^{ε}-palmitoyl Lys^{B28} Pro^{B29} human insulin, B30-N^{ε}-myristoyl-Thr^{B29}Lys^{B30} human insulin, B30-N^{ε}-palmitoyl-Thr^{B29}Lys^{B30} human insulin, B29-N^{ε}-(N-palmitoyl-γ-glutamyl)-des(B30) human insulin, B29-N^{ε}-(N-lithocholyl-γ-glutamyl)-des(B30) human insulin, B29-N^{ε}-(ω-carboxyheptadecanoyl)-des(B30) human insulin and B29-N^{ε}-(ω-carboxyheptadecanoyl) human insulin.

In a preferred embodiment the powder formulation of the present invention comprises an insulin derivative as well as human insulin or an analogue thereof.

However, human insulin is the most preferred insulin to be used in the formulation of the present invention.

In a particular embodiment of the present invention the solution of step a) further comprises zinc, preferably in an amount corresponding to 2 Zn atoms/insulin hexamer to 12 Zn atoms/insulin hexamer, more preferably 2 Zn atoms/insulin hexamer to 10 Zn atoms/insulin hexamer or 4 Zn atoms/insulin hexamer to 12 Zn atoms/insulin hexamer, still more preferably 2 Zn atoms/insulin hexamer to 5 Zn atoms/insulin hexamer.

Particular good results are obtained when the precipitation in step c) is performed by mixing insulin and the enhancer before adding the preferred amount of zinc. Moreover, particular good results are obtained when the precipitation in step c) is performed essentially without evaporation of the solution.

Furthermore, step c) is preferably carried out keeping the preparation at rest but essentially the same result is obtained under slight agitation.

In the process of the invention, the temperature during precipitation is preferably kept in the range of 0°C to 32°C, preferably 20°C to 32°C.

The employed molar ratio of insulin to enhancer is preferably 9:1 to 1:9, more preferably between 5:1 to 1:5, and still more preferably between 3:1 to 1:3.

The acidic solution of step a) preferably has a pH value in the range of 3.0 - 3.9.

In a preferred embodiment the solution of step a) contains a phenolic compound, preferably in an amount corresponding to at least 3 molecules of a phenolic compound/insulin hexamer. The phenolic compound is preferably m-cresol or phenol, or a mixture thereof.

The process of the invention is preferably carried out so as to obtain a substantially crystalline product, i.e. a product in which at least 50% by weight, preferably at least 75% by weight, more preferably at least 90% by weight, of the particles are crystalline.

The powder formulations obtained by the process of the present invention may optionally be combined with a carrier or excipient generally accepted as suitable for pulmonary administration. The purpose of adding a carrier or excipient may be as a bulking agent, stabilizing agent or an agent improving the flowing properties.

Suitable carrier agents include 1) carbohydrates, e.g. monosaccharides such as fructose, galactose, glucose, sorbose, and the like; 2) disaccharides, such as lactose, trehalose and the like; 3) polysaccharides, such as raffinose, maltodextrins, dextrans, and the like; 4) alditols, such as mannitol, xylitol, and the like; 5) inorganic salts, such as sodium chloride, and the like; 6) organic salt, such as sodium citrate, sodium ascorbate, and the like. A preferred group of carriers includes trehalose, raffinose, mannitol, sorbitol, xylitol, inositol, sucrose, sodium chloride and sodium citrate.

This invention is further illustrated by the following examples which, however, are not to be construed as limiting.

### EXAMPLE I

625.9 mg human insulin was dissolved in water by adding 2N HCI resulting in a pH = 3.6-3.7. 125 µL 4 % Zinc chloride solution was added to the insulin solution while mixing. Water was added to 25 mL. 1g sodium taurocholate was dissolved in 10 mL water. To 16 mL of the insulin solution was then added 4 mL of the taurocholate solution while mixing. Water ad 100 mL was finally added while mixing. The preparation with the spontaneous amorphous precipitate was divided in 7 beakers with 10 mL in each. The pH was adjusted to 4.5, 5.0, 5.5, 6.0, 6.1, 6.5, 7.0 and 7.4 while mixing. After standing at rest for approximately 16 hours at 20°C - 25°C, crystals were formed in all preparations.

An aliquot of each preparation elucidates almost complete crystalline state of the particles as determined under a polarized light microscope. The size of the individual crystals was determined to 1µm - 5µm.

The supernatant was carefully removed from each of the preparations and the remaining wet crystalline fraction was dried by placing in a vacuumdryer for approximately 5 hours.

The dry insulin powders were analyzed by RP-HPLC for the content of human insulin and sodium taurocholate and the results showed a proportion of human insulin and sodium taurocholate of 3:1 to 7:1 depending on the actual pH value .

### EXAMPLE II

625.9 mg human insulin was dissolved in water by adding 2N HCI resulting in a pH= 3.6-3.7. Water was added to 25 mL. 1g sodium taurocholate was dissolved in 10 mL water. The insulin solution was divided in 6 beakers with 4 mL in each. A 0.4 % Zinc chloride solution was added to the insulin solutions while mixing in an increasing amount: 81 µL, 123 µL, 164µL, 205µL, 285µL and 410µL. To each of the solutions were then added 1 mL of the taurocholate solution while mixing. Water ad 25 mL was finally added while mixing. The pH was adjusted to 6.1 while mixing. Spontaneously, an amorphous precipitate was formed in each of the preparations. After standing at rest for approximately 16 hours at 20°C - 25°C, crystals were formed in all preparations.

An aliquot of each preparation elucidates almost complete crystalline state of the particles as determined under a polarized light microscope. The size of the individual crystals was determined to 1µ - 5µ.

The supernatant was carefully removed from each of the preparations and the remaining wet crystalline fraction was dried by placing in a vacuumdryer for approximately 5 hours.

The dry insulin powders were analyzed by RP-HPLC for the content of human insulin and sodium taurocholate and the results showed a proportion of human insulin and sodium taurocholate of 4:1 to 5:1 depending on the content of zinc.

### EXAMPLE III

625,3 mg human insulin was dissolved in water by adding 2N HCI resulting in a pH= 3.6-3.7. 125µL 4 % Zinc chloride solution was added to the insulin solution while mixing. Water was added to 25 mL. 1 g sodium taurocholate was dissolved in 10 mL water. The insulin solution was divided in 4 beakers with 1.6 mL in each. To each of the beakers were added 400 µL of taurocholate solution while mixing. A sodium chloride solution (100 mg/mL) was added while mixing in an increasing amount: 0 µL, 58µL, 116µL and 232µL.Water ad 10 mL was finally added while mixing. The pH was adjusted to 6.1 while mixing. An aliquot of each preparation elucidates 50% to 80% crystalline state of the particles as determined under a polarized light microscope. The size of the individual crystals was determined to 1µ - 5µ.

The dry insulin powders were analyzed for the content of human insulin and sodium taurocholate and the results showed a proportion of human insulin and sodium taurocholate of 3:1 in all the preparations

### EXAMPLE IV

2.5 g human insulin was dissolved in water by adding 2N HCI resulting in a pH= 3.6 - 3.7. 500 µL 4 % Zinc chloride solution was added to the insulin solution while mixing. Water was added to 100 mL. 2.5 g sodium taurocholate was dissolved in 25 mL water. The insulin solution was divided in 9 beakers with 8 mL in each. To 3 insulin solutions (group 1) were added 2 mL, to the next 3 insulin solutions (group 2) were added 2.25 mL and to the last 3 insulin solutions (group 3) were added 2.50 mL of the taurocholate solution while mixing. In each of the 3 groups, a sodium chloride solution (100 mg/mL) was added in increasing amounts: 0 µL, 290 µL and 1160 µL. Water ad 50 mL was finally added while mixing. The pH was adjusted to 6.1 while mixing. Spontaneously, an amorphous precipitate was formed in each of the preparations. After standing at rest for approximately 16 hours at 20°C - 25°C, crystals were formed in all preparations.

An aliquot of each preparation elucidates almost complete crystalline state of the particles with no sodium chloride added while the preparations with sodium chloride elucidate approximately 50 % to 80 % crystalline state as determined under a polarized light microscope. The size of the individual crystals was determined to 1µ - 5µ.

The supernatant was carefully removed from each of the preparations and the remaining wet crystalline fraction was dried by placing in a vacuum dryer for approximately 5 hours.

The dry insulin powders were analyzed for the content of human insulin and sodium taurocholate and the results showed a proportion of human insulin and sodium taurocholate of 6:1 to 3:1 in the preparations.

## Claims

1. A process for the preparation of a therapeutic powder formulation comprising particles composed of insulin or an analogue or derivative thereof and an enhancer which enhances the absorption of insulin in the lower respiratory tract, comprising the steps of:
a) providing an acidic aqueous solution comprising insulin or an analogue or derivative thereof, an enhancer and optionally zinc;
b) adjusting the pH value to 4.5 to 7.4, preferably 4.5 to 7, more preferably 4.5 to 6.5, still more preferably 5.5 to 6.2, most preferably 5.5 to 6.1;
c) precipitating, essentially without evaporation of the solution, a product comprising insulin or an analogue or derivative thereof, an enhancer and optionally zinc; and
d) removing the water.

2. A process according to claim 1 wherein the enhancer is a surfactant.

3. A process according to claim 2 wherein the surfactant is a salt of a fatty acid, a bile salt or a phospholipid, preferably a bile salt.

4. A process according to claim 3 wherein the surfactant is a salt of taurocholate, preferably sodium taurocholate.

5. A process according to anyone of the preceding claims wherein the solution of step a) contains zinc in an amount corresponding to 2 Zn atoms/insulin hexamer to 12 Zn atoms/insulin hexamer, preferably 4 Zn atoms/insulin hexamer to 12 Zn atoms/insulin hexamer

6. A process according to claim 5 wherein the solution of step a) contains zinc in an amount corresponding to 2 Zn atoms/insulin hexamer to 10 Zn atoms/insulin hexamer, preferably 2 Zn atoms/insulin hexamer to 5 Zn atoms/insulin hexamer.

7. A process according to anyone of the preceding claims wherein the solution of step a) has a pH value in the range of 3.0 - 3.9.

8. A process according to anyone of the preceding claims wherein the solution of step a) further comprises a phenolic compound, preferably in an amount corresponding to at least 3 molecules of a phenolic compound/insulin hexamer.

9. A process according to claim 8 wherein the solution of step a) comprises m-cresol or phenol, or a mixture thereof.

10. A process according to anyone of the preceding claims wherein the precipitation in step c) is performed under slight agitation.

11. A process according to anyone of the preceding claims wherein the removal of water in step d) is carried out using vacuum evaporation.

12. A process according to anyone of the preceding claims which further comprises micronizing the product of step d).

13. A process according to anyone of the preceding claims, wherein the temperature during precipitation is kept in the range of 0°C to 32°C, preferably 20°C to 32°C.

14. A process according to anyone of the preceding claims, wherein the molar ratio of insulin to enhancer is 9:1 to 1:9, preferably between 5:1 to 1:5, and more preferably between 3:1 to 1:3.

15. A therapeutic powder formulation obtainable by a process according to any one of the preceding claims.

## Patentansprüche

1. Verfahren zur Herstellung einer therapeutischen Pulverformulierung umfassend Partikel bestehend aus Insulin oder einem Analog oder Derivat davon und einem Verstärker, der die Absorption von Insulin in dem unteren Atemtrakt verstärkt, umfassend die Schritte:
a) Bereitstellen einer sauren wässrigen Lösung umfassend Insulin oder ein Analog oder Derivat davon, einen Verstärker und wahlweise Zink;
b) Einstellen des pH-Wertes bei 4,5 bis 7,4, vorzugsweise 4,5 bis 7, weiter bevorzugt 4,5 bis 6,5, noch weiter bevorzugt 5,5 bis 6,2, am meisten bevorzugt 5,5 bis 6,1;
c) Präzipitieren, im wesentlichen ohne Verdampfen der Lösung, eines Produkts umfassend Insulin oder ein Analog oder Derivat davon, eines Verstärker und wahlweise Zink; und
d) Entfernen des Wassers.

2. Verfahren nach Anspruch 1, wobei der Verstärker ein oberflächenaktiver Stoff ist.

3. Verfahren nach Anspruch 2, wobei der oberflächenaktive Stoff ein Salz oder eine Fettsäure, ein Gallensalz oder ein Phospholipid, vorzugsweise ein Gallensalz ist.

4. Verfahren nach Anspruch 3, wobei der oberflächenaktive Stoff ein Salz von Taurocholat, vorzugsweise Natriumtaurocholat ist.

5. Verfahren nach einem der vorhergehenden Ansprüche, wobei die Lösung aus Schritt a) Zink in einer Menge entsprechend 2 Zn Atome/Insulinhexamer bis 12 Zn Atome/Insulinhexamer, vorzugsweise 4 Zn Atome/Insulinhexamer bis 12 Zn Atome/Insulinhexamer aufweist.

6. Verfahren nach Anspruch 5, wobei die Lösung aus Schritt a) Zink in einer Menge entsprechend 2 Zn Atome/Insulinhexamer bis 10 Zn Atomen/Insulinhexamer, vorzugsweise 2 Zn Atome/Insulinhexamer bis 5 Zn Atome/Insulinhexamer aufweist.

7. Verfahren nach einem der vorhergehenden Ansprüche, wobei die Lösung aus Schritt a) einen pH-Wert im Bereich von 3,0 - 3,9 aufweist.

8. Verfahren nach einem der vorhergehenden Ansprüche, wobei die Lösung aus Schritt a) weiterhin eine phenolische Verbindung, vorzugsweise in einer Menge entsprechend wenigstens 3 Moleküle einer phenolischen Verbindung/Insulinhexamer aufweist.

9. Verfahren nach Anspruch 8, wobei die Lösung aus Schritt a) m-Cresol oder Phenol, oder eine Mischung davon aufweist.

10. Verfahren nach einem der vorhergehenden Ansprüche, wobei die Präzipitation in Schritt c) unter leichtem Rühren durchgeführt wird.

11. Verfahren nach einem der vorhergehenden Ansprüche, wobei das Entfernen von Wasser in Schritt d) unter Verwendung von Vakuumverdampfung ausgeführt wird.

12. Verfahren nach einem der vorhergehenden Ansprüche, das weiterhin Zerkleinern des Produkts aus Schritt d) aufweist.

13. Verfahren nach einem der vorhergehenden Ansprüche, wobei die Temperatur während der Präzipitation im Bereich von 0°C bis 32°C, vorzugsweise 20°C bis 32°C gehalten wird.

14. Verfahren nach einem der vorhergehenden Ansprüche, wobei das molare Verhältnis von Insulin zu Verstärker 9:1 bis 1:9, vorzugsweise zwischen 5:1 bis 1:5, und weiter bevorzugt zwischen 3:1 bis 1:3 beträgt.

15. Eine therapeutische Pulverformulierung erhältlich durch ein Verfahren gemäß einem der vorhergehenden Ansprüche.

## Revendications

1. Un procédé pour la préparation d'une formulation de poudre thérapeutique comprenant des particules composées d'insuline ou d'un analogue ou d'un dérivé de celle-ci et d'un activateur qui active l'absorption d'insuline dans la voie respiratoire inférieure, comprenant les étapes consistant à :
a) prévoir une solution aqueuse acide comprenant de l'insuline ou un analogue ou un dérivé de celle-ci, un activateur et éventuellement du zinc ;
b) ajuster la valeur de pH de 4,5 à 7,4, de préférence de 4,5 à 7, notamment de 4,5 à 6,5, mieux encore de 5,5 à 6,2 et tout particulièrement de 5,5 à 6.1 ;
c) précipiter essentiellement sans évaporation de la solution un produit comprenant de l'insuline ou un analogue ou un dérivé de celle-ci, un activateur et éventuellement du zinc ; et
d) éliminer l'eau.

2. Un procédé selon la revendication 1, dans lequel l'activateur est un tensioactif.

3. Un procédé selon la revendication 2, dans lequel le tensioactif est un sel d'un acide gras, un sel de la bile ou un phospholipide, de préférence un sel de la bile.

4. Un procédé selon la revendication 3, dans lequel le tensioactif est un taurocholate, de préférence du taurocholate de sodium.

5. Un procédé selon l'une quelconque des revendications précédentes, dans lequel la solution de l'étape a) renferme du zinc selon une quantité correspondant à 2 atomes de Zn/hexamère d'insuline à 12 atomes de Zn/hexamère d'insuline, de préférence de 4 atomes de Zn/hexamère d'insuline à 12 atomes de Zn/hexamère d'insuline.

6. Un procédé selon la revendication 5, dans lequel la solution de l'étape a) renferme du zinc selon une quantité correspondant à 2 atomes de Zn/hexamère d'insuline à 10 atomes de Zn/hexamère d'insuline, de préférence de 2 atomes de Zn/hexamère d'insuline à 5 atomes de Zn/hexamère d'insuline.

7. Un procédé selon l'une quelconque des revendications précédentes, dans lequel la solution de l'étape a) a une valeur de pH comprise dans la gamme de 3,0 à 3,9.

8. Un procédé selon l'une quelconque des revendications précédentes, dans lequel la solution de l'étape a) comprend, en outre, un composé phénolique, de préférence selon une quantité correspondant à au moins 3 molécules d'un composé phénolique/hexamère d'insuline.

9. Un procédé selon la revendication 8, dans lequel la solution de l'étape a) comprend du m-crésol ou du phénol ou un mélange de ceux-ci.

10. Un procédé selon l'une quelconque des revendications précédentes, dans lequel la précipitation dans l'étape c) est réalisée sous une légère agitation.

11. Un procédé selon l'une quelconque des revendications précédentes, dans lequel l'élimination de l'eau dans l'étape d) est mise en oeuvre en utilisant une évaporation sous vide.

12. Un procédé selon l'une quelconque des revendications précédentes qui comprend, en outre, la micronisation du produit de l'étape d).

13. Un procédé selon l'une quelconque des revendications précédentes, dans lequel la température durant la précipitation est maintenue dans la gamme de 0°C à 32°C, de préférence de 20°C à 32°C.

14. Un procédé selon l'une quelconque des revendications précédentes, dans lequel le rapport molaire de l'insuline à l'activateur est de 9:1 à 1:9, de préférence il est compris entre 5:1 et 1:5 et mieux encore entre 3:1 et 1:3.

15. Une formulation de poudre thérapeutique apte à être obtenue par un procédé selon l'une quelconque des revendications précédentes.
